# EUROPÄISCHE PATENTANMELDUNG

(11) **EP 4 570 273 A1**
(43) Veröffentlichungstag der Anmeldung: **18.06.2025**
(21) Anmeldenummer: 23216938.3
(22) Anmeldetag: 14.12.2023
(51) Int. Cl.: A61L 2/20, A61B 50/30, B32B 27/00, B65B 55/00

(54) **VERFAHREN ZUM TRANSFER EINES GEBINDES ZUR VERARBEITUNG IN EINER PROZESSKAMMER EINES CONTAINMENTS UNTER ASEPTISCHEN BEDINGUNGEN**

(71) Anmelder: SKAN AG, 4123 Allschwil (CH)
(72) Erfinder: Lehmann, Frank Martin, 4102 Binningen (CH); Sommerhalder, Matthias, 4112 Bättwil (CH)
(74) Vertreter: Ullrich, Gerhard

(57) **Zusammenfassung**

Das konzipierte Verfahren betrifft den Transfer eines Gebindes **(5)** zur Verarbeitung in der Prozesskammer **(90)** eines Containments **(9)** unter aseptischen Bedingungen in zwei Ausführungsformen. Ein einzelnes Gebinde **(5)** umfasst ein Behältnis **(1)** mit einem aseptischen Innenvolumen (**10**) und zumindest einem ersten Durchlass **(12),** der verschlossen ist. Das Behältnis **(1)** ist z.B. ein wannenförmiges Tub mit darin angeordneten Artikeln, wie Phiolen, Vials oder Spritzen. Die mit der Erfindung gelöste Aufgabe besteht darin, dass das während einer unerlässlichen Dekontaminationsphase auf das Äussere des Gebindes **(5)** geleitete Dekontaminationsmittel nur mehr in einer minimalen, akzeptablen Konzentration in das Innenvolumen (**10**) des Behältnisses **(1)** gelangt. Hierzu wird in einem Verfahrensschritt an einer ansonsten semipermeablen Abdeckung **(2),** welche den zumindest einen ersten Durchlass **(12)** oder einen zweiten Durchlass **(12')** nicht gasdicht verschliesst, eine wesentlich dichtere Schutzschicht **(4)** vorgesehen. Zur Durchführung des Verfahrens dient eine Verkettung aus Reinraum **(6),** dem anschliessenden RABS **(7),** der folgenden Transfereinrichtung **(8)** und dem damit andererseits verbundenen Containment **(9).**

## Beschreibung

### Anwendungsgebiet der Erfindung

Die vorliegende Erfindung betrifft ein Verfahren zum Transfer eines Gebindes zur Verarbeitung in der Prozesskammer eines Containments unter aseptischen Bedingungen in zwei Ausführungsformen. Ein einzelnes Gebinde umfasst ein Behältnis mit einem aseptischen Innenvolumen und zumindest einem ersten Durchlass, der verschlossen ist. Das Behältnis ist z.B. ein wannenförmiges Tub oder Tray mit darin angeordneten Artikeln, wie Phiolen, Vials oder Spritzen. Hierbei können die Artikel in einem in das Behältnis eingesetzten Nest positioniert sein.

### Stand der Technik

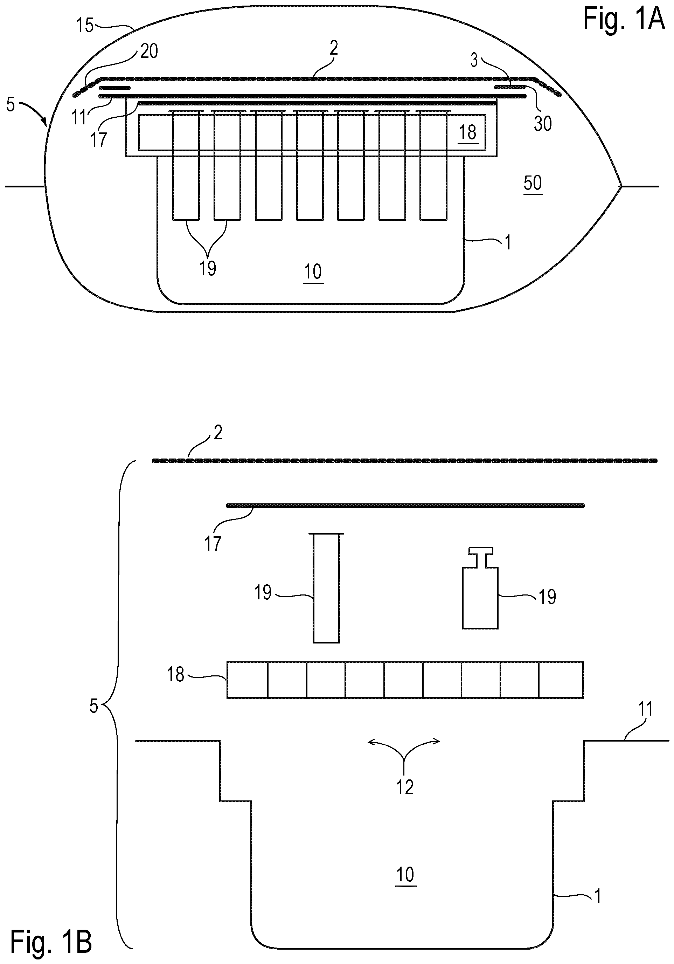

Die Figuren 1A und 1B illustrieren den vorbekannten Stand der Technik.

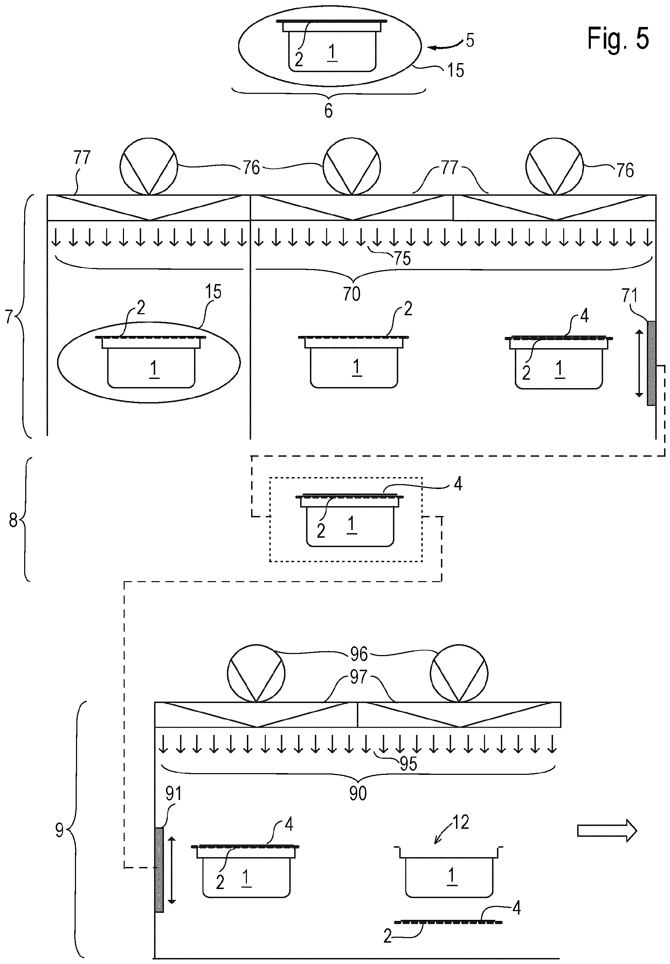

Ein einzelnes Gebinde **5** umfasst:
- ein Behältnis **1** mit einem aseptischen Innenvolumen **10** und einem ersten Durchlass **12,** der mit einer Abdeckung **2** verschlossen ist;
- ein im Innenvolumen **10** gelagertes Nest **18,** in dessen muldenförmigen Aufnahmekonturen die Artikel **19** stecken, welche nach Öffnen der Abdeckung **2** in der Prozesskammer **90** des Containments **9** (siehe Figur 5) zu behandeln sind;
- optional eine zwischen der Abdeckung **2** und dem Nest **18,** über den Artikeln **19** eingefügte Zwischenlage **17;** sowie
- üblicherweise eine im Anlieferungszustand das gesamte Gebinde **5** umgebende, geschlossene beutelartige Umhüllung **15,** die den Innenraum **50** steril hält.

Das Behältnis **1** ist z.B. ein wannenförmiges Tub. Die Artikel **19** sind insbesondere Phiolen, Vials oder Spritzen. Von der Abdeckung **2** und der Umhüllung **15** bestehen vorzugsweise zumindest Flächenanteile aus einem semipermeablen Vliesgewebe, wie Tyvek^{®}. Die Abdeckung **2** ist typischerweise auf dem den ersten Durchlass **12** des Behältnisses **1** umlaufenden Randfläche **11** versiegelt, z.B. verklebt oder verschweisst - bildet eine erste Anhaftung **3** mit dem Rand **30** -, und bewahrt so das Innenvolumen **10** des Behältnisses **1** in sterilem Zustand.

Nach dem Auspacken des Gebindes **5** aus der Umhüllung **15** und bevor es unter aseptischen Bedingungen zur Verarbeitung in die Prozesskammer eines Containments transferiert werden kann, muss das Gebinde **5** äusserlich dekontaminiert werden. Nachteilig hierbei ist, dass bestimmte auf das Gebinde **5** einwirkende Dekontaminationsmittel durch die semipermeable Abdeckung **2** in einem unerwünscht hohen Mass in das Innenvolumen **10** des Behältnisses **1** und an dessen Inhalt gelangen und nach Öffnen der Abdeckung **2** in der Prozesskammer des Containments dort ausströmen. Dies kann, obwohl die ausströmenden Dekontaminationsreste in der Prozesskammer schnell verdünnt werden, dennoch kritisch sein. Sehr kritisch ist jedoch, wenn innerhalb der Artikel **19** Reste des gasförmigen Dekontaminationsmittels - z.B. H₂O₂ - verbleiben, die dann die Wirkung des Medikaments verändern können, wenn es in die Artikel **19** abgefüllt wird. Ferner können sich Reste des Dekontaminationsmittels in das Material der Artikel **19** und/oder in deren Verschlusselemente (z.B. Stopfen) einlagern und somit nach dem Einfüllen eines Medikaments in dieses gelangen.

### Aufgabe der Erfindung

Aufgabe der vorliegenden Erfindung ist es daher, basierend auf der von der Industrie gemäss vorbekanntem Stand der Technik bereitgestellten Gebinden, verfahrenseffiziente Massnahmen vorzuschlagen, so dass das während einer unerlässlichen Dekontaminationsphase auf das Äussere des Gebindes geleitete Dekontaminationsmittel nur mehr in einer minimalen, akzeptablen Konzentration durch die Abdeckung in das Innenvolumen des Behältnisses gelangt. Massgebliche Zielstellung ist, dass während des Verfahrensablaufs möglichst kein Dekontaminationsmittel in die zu verarbeitende Substanz, insbesondere Medikamente, gelangt.

Es geht also immer darum, dass möglichst kein Dekontaminationsmittel in das Medikament gelangt, so dass sich das Medikament nicht unerwünscht verändert.

### Übersicht über die Erfindung

Das erfindungsgemässe Verfahren zum Transfer eines Gebindes zur Verarbeitung in einer Prozesskammer eines Containments unter aseptischen Bedingungen ist in einer *ersten Ausführungsform* gekennzeichnet durch die Abfolge:
a) Bereitstellung des Gebindes zumindest umfassend ein Behältnis mit einem aseptischen Innenvolumen und einem zuoberst liegenden Durchlass, der mit einer partiell durchlässigen Abdeckung verschlossen ist;
b) Anbringen einer Schutzschicht an der Abdeckung, wobei die Schutzschicht:
   ba) im Vergleich zur Abdeckung weniger für das während einer Dekontaminationsphase auf das Gebinde eingeleitete Dekontaminationsmittel durchlässig ist;
   bb) sich zumindest partiell oder gänzlich über die Abdeckung erstreckt; und
   bc) dazu bestimmt ist, dass das während der Dekontaminationsphase auf das Gebinde eingeleitete Dekontaminationsmittel nur mehr in einer minimalen, akzeptablen Konzentration durch die Abdeckung in das Innenvolumen gelangt;
c) Durchführen einer Dekontaminationsphase mit dem mit der Abdeckung und der Schutzschicht versehenen Gebindes in einer Transfereinrichtung;
d) Überführen des mit der Abdeckung und der Schutzschicht dekontaminierten Gebindes in die Prozesskammer des Containments; und
e) in der Prozesskammer des Containments Entfernen der Abdeckung und Schutzschicht vom Gebinde, so dass der Durchlass zum Innenvolumen des Behältnisses geöffnet ist und die weitere Verarbeitung erfolgen kann.

Nachfolgend werden untergeordnete Merkmale des erfindungsgemässen Verfahrens in dessen *erster Ausführungsform* definiert: Die Abdeckung und die Schutzschicht werden als Verbund zusammen vom Durchlass entfernt.

Die Schutzschicht wird als separates Mittel auf der Abdeckung aufgebracht und/oder wird durch Einwirken auf die Abdeckung mit daran entstehender Strukturänderung gebildet, welche als Schutzschicht wirkt. Das Einwirken auf die Abdeckung zur Bildung der Schutzschicht geschieht z.B. thermisch oder mittels Bestrahlung, vorzugsweise mit UV-Strahlung oder Ultraschall.

Die Abdeckung ist eine semipermeable Folie, z.B. Tyvek^{®}, welche auf einer den Durchlass umlaufenden Randfläche des Behältnisses mittels einer ersten Anhaftung fixiert ist, und die Schutzschicht ist in Gestalt eines separaten Mittels als Folie beschaffen und flächig mit der Abdeckung mittels einer zweiten Anhaftung verbunden. Die Erstreckung der Schutzschicht endet vor dem äusseren Rand der Abdeckung.

Während der Dekontaminationsphase kann Dekontaminationsmittel durch eine äussere Randzone der Abdeckung, welche nicht von der Schutzschicht abgeschirmt ist, auch zu einem Rand der ersten Anhaftung und auf eine Randfläche des Behältnisses gelangen. Als Dekontaminationsmittel wird Wasserstoffperoxid (H₂O₂) oder Stickstoffdioxid (NO₂) oder ein Begasungsmittel in fachgemässer Konzentration verwendet.

Während der Bereitstellung des Gebindes liegt das Behältnis mit seinem aseptischen Innenvolumen und der Abdeckung, welche den Durchlass verschliesst, in einer beutelartigen Umhüllung. Die Bereitstellung des Gebindes erfolgt in einem Reinraum, vorzugsweise mit Reinraumklasse Grade C (gemäss GMP Annex 1: Good Manufacturing Practice), äquivalent zu Klasse 7 (gemäss ISO-Norm 14644-1).

Das Gebinde wird vom Reinraum in ein RABS (Restricted Access Barrier System) transferiert und hier wird die das Gebinde umgebende Umhüllung entfernt, wobei das RABS vorzugsweise die Reinraumklasse Grade A für Supply (gemäss GMP Annex 1: Good Manufacturing Practice), äquivalent zu Klasse 5 (gemäss ISO-Norm 14644-1) aufweist. Die Transfereinrichtung ist einerseits mit dem RABS und andererseits mit dem Containment verkettet.

Das erfindungsgemässe Verfahren zum Transfer eines Gebindes zur Verarbeitung in einer Prozesskammer eines Containments unter aseptischen Bedingungen ist in einer *zweiten Ausführungsform* gekennzeichnet durch die Abfolge:
a) Bereitstellung des Gebindes zumindest umfassend ein Behältnis mit einem aseptischen Innenvolumen, wobei das Behältnis ausweist:
   aa) einen zuoberst liegenden ersten Durchlass, der mit einer undurchlässigen Überdeckung verschlossen ist; und
   ab) einen am Boden oder seitlich des Behältnisses liegenden zweiten Durchlass, der mit einer partiell durchlässigen Abdeckung verschlossen ist;
b) Anbringen einer Schutzschicht an der Abdeckung, wobei die Schutzschicht:
   ba) im Vergleich zur Abdeckung weniger für das während einer Dekontaminationsphase auf das Gebinde eingeleitete Dekontaminationsmittel durchlässig ist;
   bb) sich zumindest partiell oder gänzlich über die Abdeckung erstreckt; und
   bc) dazu bestimmt ist, dass das während der Dekontaminationsphase auf das Gebinde eingeleitete Dekontaminationsmittel nur mehr in einer minimalen, akzeptablen Konzentration durch die Abdeckung in das Innenvolumen gelangt;
c) Durchführen einer Dekontaminationsphase mit dem mit der Abdeckung und der Schutzschicht versehenen Gebinde in einer Transfereinrichtung;
d) Überführen des mit der Abdeckung und der Schutzschicht dekontaminierten Gebindes in die Prozesskammer des Containments; und
e) in der Prozesskammer des Containments Entfernen der Abdeckung vom Gebinde, so dass der erste Durchlass zum Innenvolumen des Behältnisses geöffnet ist und die weitere Verarbeitung erfolgen kann.

Nachfolgend werden untergeordnete Merkmale des erfindungsgemässen Verfahrens in dessen *zweiter Ausführungsform* definiert: Die Schutzschicht wird als separates Mittel auf der Abdeckung aufgebracht und/oder wird durch Einwirken auf die Abdeckung mit daran entstehender Strukturänderung gebildet, welche als Schutzschicht wirkt. Das Einwirken auf die Abdeckung zur Bildung der Schutzschicht geschieht z.B. thermisch oder mittels Bestrahlung, vorzugsweise mit UV-Strahlung oder Ultraschall.

Die Abdeckung ist eine semipermeable Folie, z.B. Tyvek^{®}, welche auf einer den zweiten Durchlass umlaufenden Randfläche des Behältnisses mittels einer ersten Anhaftung fixiert ist, und die Schutzschicht ist in Gestalt eines separaten Mittels als Folie beschaffen und flächig mit der Abdeckung mittels einer zweiten Anhaftung verbunden. Die Erstreckung der Schutzschicht endet vor dem äusseren Rand der Abdeckung.

Während der Dekontaminationsphase kann Dekontaminationsmittel durch eine äussere Randzone der Abdeckung, welche nicht von der Schutzschicht abgeschirmt ist, auch zu einem Rand der ersten Anhaftung und auf die den zweiten Durchlass umlaufende Randfläche des Behältnisses gelangen. Als Dekontaminationsmittel wird Wasserstoffperoxid (H₂O₂) oder Stickstoffdioxid (NO₂) oder ein Begasungsmittel in fachgemässer Konzentration verwendet.

Während der Bereitstellung des Gebindes liegt das Behältnis (1) mit seinem aseptischen Innenvolumen und der Abdeckung, welche den zweiten Durchlass verschliesst, und der Überdeckung, welche den ersten Durchlass verschliesst, in einer beutelartigen Umhüllung. Die Bereitstellung des Gebindes erfolgt in einem Reinraum, vorzugsweise mit Reinraumklasse Grade C (gemäss GMP Annex 1: Good Manufacturing Practice), äquivalent zu Klasse 7 (gemäss ISO-Norm 14644-1).

Das Gebinde wird vom Reinraum in ein RABS (Restricted Access Barrier System) transferiert und hier wird die das Gebinde umgebende Umhüllung entfernt, wobei das RABS vorzugsweise die Reinraumklasse Grade A für Supply (gemäss GMP Annex 1: Good Manufacturing Practice), äquivalent zu Klasse 5 (gemäss ISO-Norm 14644-1) aufweist. Die Transfereinrichtung ist einerseits mit dem RABS und andererseits mit dem Containment verkettet.

### Kurzbeschreibung der beigefügten Zeichnungen

Es zeigen:
- Figur 1A -: ein mit dem erfindungsgemässen Verfahren *erster Ausführungsform* zu verarbeitendes Gebinde mit geschlossener Umhüllung;
- Figur 1B -: das Gebinde gemäss Figur 1A ohne die Umhüllung in Explosivansicht;
- Figur 2A -: das Behältnis aus Figur 1A, ohne Inhalt und mit einer Abdeckung über einem zuoberst am Behältnis vorhandenen ersten Durchlass, als Prinzipdarstellung;
- Figur 2B -: das Behältnis gemäss Figur 2A, zusätzlich mit einer Schutzschicht versehen, in erster Gestaltung;
- Figur 2C -: das Behältnis gemäss Figur 2A, zusätzlich mit einer Schutzschicht versehen, in zweiter Gestaltung;
- Figur 3A -: die Anordnung aus Figur 2B, mit der Randfläche des Behältnisses, der Abdeckung, Verklebungen und Schutzschicht, als Detailansicht;
- Figur 3B -: die Anordnung aus Figur 2C, mit der Randfläche des Behältnisses, der Abdeckung, Verklebungen und Schutzschicht, als Detailansicht;
- Figur 4A -: das Behältnis aus Figur 1A mit einer Abdeckung über einem am Boden des Behältnisses vorhandenen zweiten Durchlass, für das Transferverfahren *zweiter Ausführungsform,* als Prinzipdarstellung;
- Figur 4B -: das Behältnis aus Figur 1A mit einer Abdeckung über einem seitlich am Behältnis vorhanden zweiten Durchlass, für das Transferverfahren *zweiter Ausführungsform,* als Prinzipdarstellung; und
- Figur 5 -: die Verkettung von Reinraum für die Bereitstellung des Gebindes, dem anschliessenden RABS, der folgenden Transfereinrichtung und dem damit andererseits verbundenen Containment zur Durchführung des erfindungsgemässen Verfahrens *erster Ausführungsform.*

### Ausführungsbeispiel

Mit Bezug auf die beiliegenden Zeichnungen erfolgt nachstehend die detaillierte Beschreibung des erfindungsgemässen Verfahrens zum Transfer eines Gebindes zur Verarbeitung in einer Prozesskammer eines Containments unter aseptischen Bedingungen in zwei *Ausführungsformen.*

### Figur 2A

Am Start des Transferverfahrens in seiner *ersten Ausführungsform* steht nach Entfernung der zumeist vorhandenen Umhüllung **15** das Behältnis **1** aus Figur 1A, mit der partiell durchlässigen Abdeckung **2** über dem zuoberst am Behältnis **1** vorhandenen ersten Durchlass **12** bereit. Vereinfacht ist das Behältnis **1** mit seinem Innenvolumen **10** ohne Inhalt dargestellt. Die Abdeckung **2** ist eine semipermeable Folie, z.B. Tyvek^{®}, und auf der Randfläche **11** mittels einer ersten Anhaftung **3** als Verklebung oder Verschweissung fixiert, wobei der äussere Rand **30** der ersten Anhaftung **3** sich bis an die Aussenkante der Randfläche **11** erstreckt und die Randzone **20** der Abdeckung **2** den Rand **30** der ersten Anhaftung **3** und die Aussenkante der Randfläche **11** überragt.

### Figuren 2B und 3A

Im nächsten Schritt des Transferverfahrens wird eine Schutzschicht **4** an der Abdeckung **2** angebracht. Im Vergleich zur Abdeckung **2** ist die Schutzschicht **4** weniger für das während einer späteren Dekontaminationsphase auf das Gebinde **5** eingeleitete Dekontaminationsmittel durchlässig, so dass das Dekontaminationsmittel nur mehr in einer minimalen, akzeptablen Konzentration durch die Abdeckung **2** in das Innenvolumen **10** gelangt. In der gezeigten ersten Gestaltung erstreckt sich die Schutzschicht **4** gänzlich über die Abdeckung **2,** abgesehen von ihrer überhängenden Randzone **20,** und hat die Gestalt einer Folie als separates Mittel, welches flächig mit der Abdeckung **2** mittels der zweiten Anhaftung **40** verbunden ist. Alternativ kann die Schutzschicht **4** durch strukturänderndes Einwirken auf die Abdeckung **2** gebildet werden, welche dann als Schutzschicht **4** wirkt. Das Einwirken auf die Abdeckung **2** zur Bildung der Schutzschicht **4** kann thermisch oder mittels Bestrahlung, vorzugsweise mit UV-Strahlung oder Ultraschall, geschehen.

### Figuren 2C und 3B

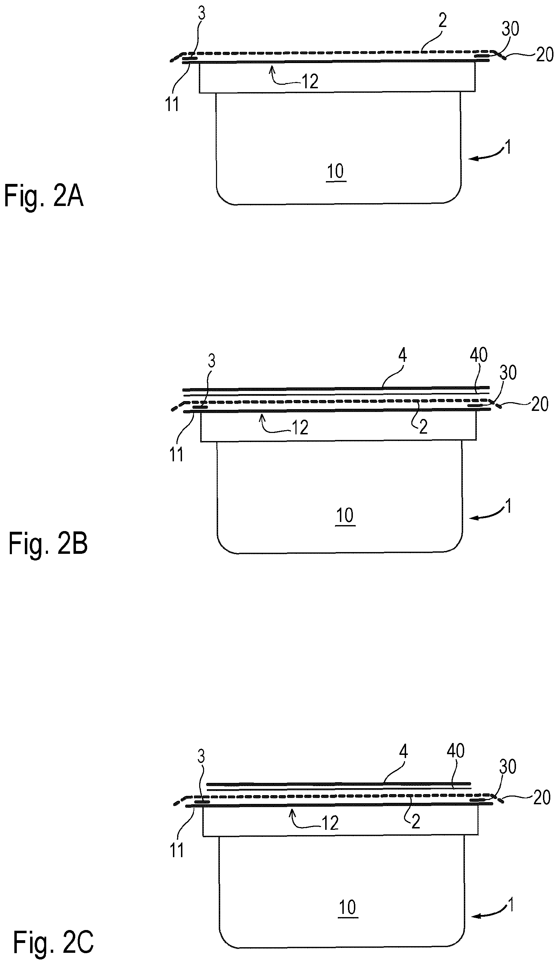

Im Unterschied zur Figur 2B endet die Schutzschicht **4** jetzt, in der zweiten Gestaltung, bereits vor der überhängenden Randzone **20** der Abdeckung **2.** Damit wird ermöglicht, dass während der späteren Dekontaminationsphase Dekontaminationsmittel durch eine äussere Randzone **20** der Abdeckung **2,** welche nicht von der Schutzschicht **4** abgeschirmt ist, auch zu einem Rand **30** der ersten Anhaftung **3** und auf eine Randfläche **11** des Behältnisses **1** gelangen kann.

### Figur 4A

Am Start des Transferverfahrens in seiner *zweiten Ausführungsform* steht nach Entfernung der zumeist vorhandenen Umhüllung **15** ein Behältnis **1** modifiziert zur Figur 1A bereit. Vereinfacht ist das Behältnis **1** mit seinem aseptischen Innenvolumen **10** wiederum ohne Inhalt dargestellt. Der zuoberst am Behältnis **1** vorgesehene erste Durchlass **12** ist mit der undurchlässigen Überdeckung **2'** verschlossen. Die Überdeckung **2'** ist auf der Randfläche **11** mittels einer Verklebung oder Verschweissung fixiert.

Dieses Behältnis **1** besitzt einen am Boden liegenden zweiten Durchlass **12',** der mit einer partiell durchlässigen Abdeckung **2** verschlossen ist. Die Abdeckung **2** ist eine semipermeable Folie, z.B. Tyvek^{®}, und auf der Randfläche **11'** mittels einer ersten Anhaftung **3** als Verklebung oder Verschweissung fixiert, wobei die Abdeckung **2** die erste Anhaftung **3** überragt. Im nächsten Schritt des Transferverfahrens wird eine Schutzschicht **4** an der Abdeckung **2** angebracht.

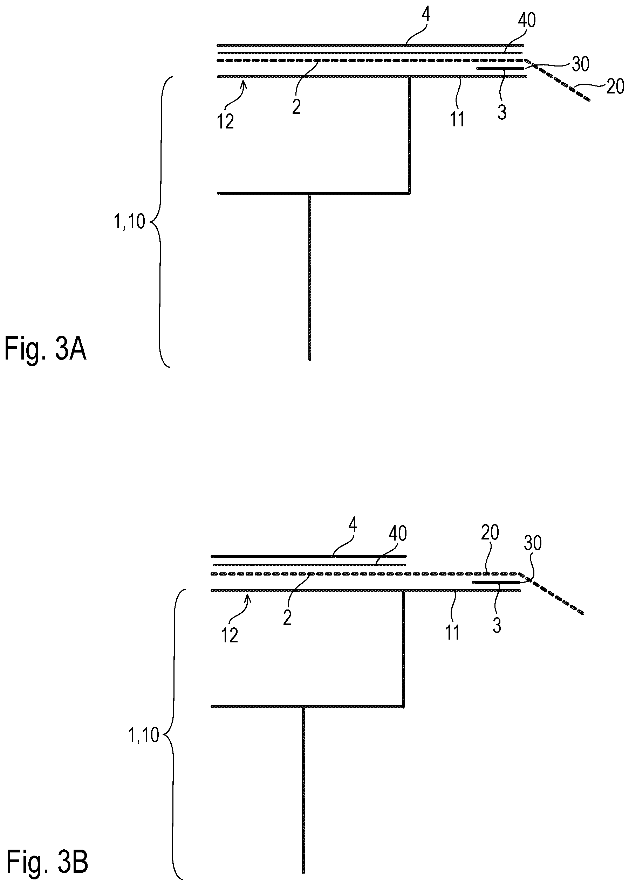

In Analogie zu Figur 2C endet die Schutzschicht **4** gemäss zweiter Gestaltung, bereits vor der äusseren Randzone **20** der Abdeckung **2.** Damit wird ermöglicht, dass während der späteren Dekontaminationsphase Dekontaminationsmittel durch die äussere Randzone **20** der Abdeckung **2,** welche nicht von der Schutzschicht **4** abgeschirmt ist, auch zu einem Rand **30** der ersten Anhaftung **3** und auf die Randfläche **11'** des Behältnisses **1** gelangen kann. Die Schutzschicht **4** hat die Gestalt einer Folie als separates Mittel, welches flächig mit der Abdeckung **2** mittels der zweiten Anhaftung **40** verbunden ist. Hinsichtlich Eigenschaften, Beschaffenheit und alternativer Bildung der Schutzschicht **4** wird auf die vorstehende Beschreibung zu Figuren 2B und 3A Bezug genommen.

### Figur 4B

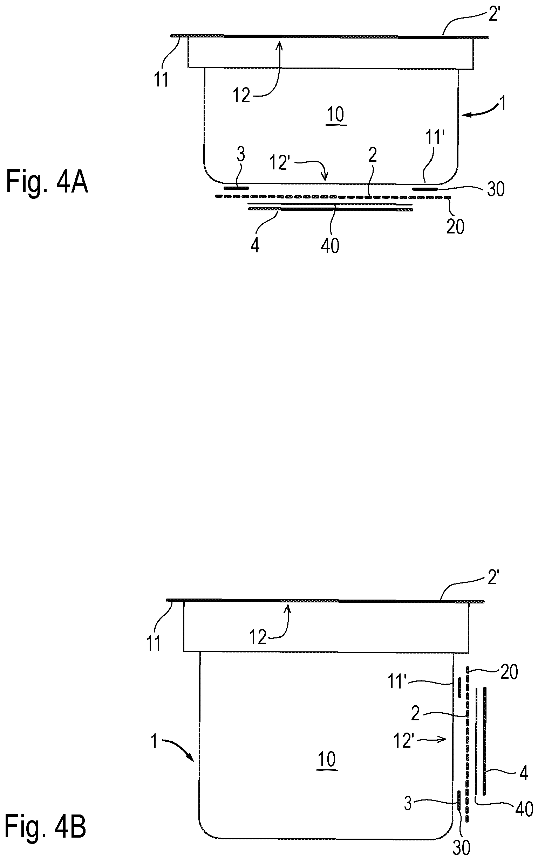

Als Variation zur Figur 4A befinden sich nun der zweite Durchlass **12',** die Abdeckung **2** mit der ersten Anhaftung **3** zur Fixierung an der Randfläche **11'** des Behältnisses **1** sowie die Schutzschicht **4** mit zugehöriger zweiter Anhaftung **40** zur Verbindung mit der Abdeckung **2,** alle seitlich am Behältnis **1.** Auch hier gilt die obige Beschreibung zu Figuren 2B und 3A bezüglich Eigenschaften, Beschaffenheit und alternativer Bildung der Schutzschicht **4.**

### Figur 5

Der Ablauf des erfindungsgemässen Verfahrens zum Transfer eines Gebindes **5** zur Verarbeitung in einer Prozesskammer **90** eines Containments **9** unter aseptischen Bedingungen, in seiner *ersten Ausführungsform,* wird anhand der dargestellten Verkettung von Reinraum **6,** anschliessendem RABS **7,** folgender Transfereinrichtung **8** und damit andererseits verbundenem Containment **9,** nachstehend beschrieben.

### Verfahrensschritt 1

Die Bereitstellung des Gebindes **5** mit der das Behältnis **1** verschliessenden semipermeablen Abdeckung **2,** welches als Einheit in der Regel steril in der Umhüllung verpackt ist, erfolgt im Reinraum **6,** vorzugsweise mit Reinraumklasse Grade C (gemäss GMP Annex 1: Good Manufacturing Practice), äquivalent zu Klasse 7 (gemäss ISO-Norm 14644-1).

### Verfahrensschritt 2

Das Gebinde **5** wird vom Reinraum **6** in die Prozesskammer **70** des RABS **7** transferiert, wo man die das Gebinde **5** umgebende Umhüllung **15** entfernt. Das RABS **7** weist vorzugsweise die Reinraumklasse Grade A für Supply (gemäss GMP Annex 1: Good Manufacturing Practice), äquivalent zu Klasse 5 (gemäss ISO-Norm 14644-1) auf. Im Plenum des RABS **7** sind je nach dessen Grösse mehrere Zuluft-Ventilatoren **76** und Zuluft-Filter **77** angeordnet, um eine die Prozesskammer **70** durchströmende gereinigte unidirektionale Verdrängungsströmung **75** zu erzeugen.

Ferner wird im RABS **7** die Abdeckung **2** am nun ausgepackten Gebinde **5** mit der Schutzschicht **4** versehen, die man als separates Mittel aufbringt und/oder durch Einwirken auf die Abdeckung **2** mit daraus resultierender Strukturänderung erzeugt, welche als Schutzschicht **4** wirkt.

### Verfahrensschritt 3

Vom RABS **7** wird das mit der Schutzschicht **4** versehene Gebinde **5** durch ein geöffnetes Ausgangs-Gate **71** in eine Transfereinrichtung **8** bewegt, in der man eine Dekontaminationsphase durchführt. Die Transfereinrichtung **8** kann insbesondere die Gestalt einer Schleuse oder eines Tunnels haben. Als Dekontaminationsmittel haben sich Wasserstoffperoxid (H₂O₂) oder Stickstoffdioxid (NO₂) oder ein Begasungsmittel in fachgemässer Konzentration bewährt.

### Verfahrensschritt 4

Aus der Transfereinrichtung **8** wird das äusserlich dekontaminierte Gebinde **5,** dessen Abdeckung **2** mit der Schutzschicht **4** versehen ist, durch ein Eingangs-Gate 91 in die Prozesskammer **90** des Containments **9** überführt. Im Plenum des Containments **9** sind je nach dessen Grösse mehrere Zuluft-Ventilatoren **96** und Zuluft-Filter **97** angeordnet, um eine die Prozesskammer **90** durchströmende gereinigte unidirektionale Verdrängungsströmung **95** zu erzeugen.

Das Containment **9** muss die Reinraumklasse Grade A (gemäss GMP Annex 1: Good Manufacturing Practice), äquivalent zu Klasse 5, erfüllen, d.h. die strengen Erfordernisse hinsichtlich Reinheit von Partikeln und Keimen in der Luft und an den Oberflächen, weswegen das Containment **9** dekontaminiert wird, zumeist mit H₂O₂.

### Verfahrensschritt 5

In der Prozesskammer **90** des Containments **9** werden vom Gebinde **5** Schutzschicht **4** und Abdeckung **2,** vorzugsweise zusammen als Verbund, entfernt, so dass der erste Durchlass **12** zum Innenvolumen **10** des Behältnisses **1** geöffnet ist und die weitere Verarbeitung erfolgen kann.

### Ablauf Transferfahren in zweiter Ausführungsform

Das Gebinde **5** gemäss Figuren 4A und 4B hat den modifizierten Aufbau mit am Boden oder seitlich des Behältnisses **1** vorgesehenem zweitem Durchlass **12',** der mit der partiell durchlässigen Abdeckung **2** und zugehöriger Schutzschicht **4** verschlossen ist. Ferner ist der zuoberst liegende erste Durchlass **12** mit einer undurchlässigen Überdeckung **2'** verschlossen. Daher muss man die Überdeckung **2'** in der Prozesskammer **90** des Containments **9** vom Behältnis **1** entfernen, um für die weitere Verarbeitung durch den dann geöffneten ersten Durchlass **12** zum Innenvolumen **10** des Behältnisses **1** für die weitere Verarbeitung zu gelangen. Abdeckung **2** und Schutzschicht **4** hingegen können am Behältnis **1** verbleiben, zusammen entsorgt oder rückgeführt werden.

## Patentansprüche

1. Verfahren zum Transfer eines Gebindes (**5**) zur Verarbeitung in einer Prozesskammer (**90**) eines Containments (**9**) unter aseptischen Bedingungen, in einer *ersten Ausführungsform,* ist **gekennzeichnet durch** die Abfolge:
a) Bereitstellung des Gebindes (**5**) zumindest umfassend ein Behältnis (**1**) mit einem aseptischen Innenvolumen (**10**) und einem zuoberst liegenden Durchlass (**12**), der mit einer partiell durchlässigen Abdeckung (**2**) verschlossen ist;
b) Anbringen einer Schutzschicht (**4**) an der Abdeckung (**2**), wobei die Schutzschicht (**4**):
ba) im Vergleich zur Abdeckung (**2**) weniger für das während einer Dekontaminationsphase auf das Gebinde (**5**) eingeleitete Dekontaminationsmittel durchlässig ist;
bb) sich zumindest partiell oder gänzlich über die Abdeckung (**2**) erstreckt; und
bc) dazu bestimmt ist, dass das während der Dekontaminationsphase auf das Gebinde (**5**) eingeleitete Dekontaminationsmittel nur mehr in einer minimalen, akzeptablen Konzentration **durch** die Abdeckung (**2**) in das Innenvolumen (**10**) gelangt;
c) Durchführen einer Dekontaminationsphase mit dem mit der Abdeckung (**2**) und der Schutzschicht (**4**) versehenen Gebindes (**5**) in einer Transfereinrichtung (**8**);
d) Überführen des mit der Abdeckung (**2**) und der Schutzschicht (**4**) dekontaminierten Gebindes (**5**) in die Prozesskammer (**90**) des Containments (**9**); und
e) in der Prozesskammer (**90**) des Containments (**9**) Entfernen der Abdeckung (**2**) und Schutzschicht (**4**) vom Gebinde (**5**), so dass der Durchlass (**12**) zum Innenvolumen (**10**) des Behältnisses (**1**) geöffnet ist und die weitere Verarbeitung erfolgen kann.

2. Verfahren nach Anspruch 1, **dadurch gekennzeichnet, dass** die Abdeckung (**2**) und die Schutzschicht (**4**) als Verbund zusammen vom Durchlass (**12**) entfernt werden.

3. Verfahren nach zumindest einem der Ansprüche 1 und 2, **dadurch gekennzeichnet, dass** die Schutzschicht (**4**):
a) als separates Mittel auf der Abdeckung (**2**) aufgebracht wird; und/oder
b) durch Einwirken auf die Abdeckung (**2**) sich daran eine Strukturänderung bildet, welche als Schutzschicht (**4**) wirkt.

4. Verfahren nach Anspruch 3, **dadurch gekennzeichnet, dass** das Einwirken auf die Abdeckung (**2**) zur Bildung der Schutzschicht (**4**) thermisch oder mittels Bestrahlung, vorzugsweise mit UV-Strahlung oder Ultraschall, geschieht.

5. Verfahren nach zumindest einem der Ansprüche 1 bis 3, **dadurch gekennzeichnet, dass**
a) die Abdeckung (**2**) eine semipermeable Folie ist, z.B. Tyvek^{®}, welche auf einer den Durchlass (**12**) umlaufenden Randfläche (**11**) des Behältnisses (**1**) mittels einer ersten Anhaftung (**3**) fixiert ist; und
b) die Schutzschicht (**4**) in Gestalt eines separaten Mittels als Folie beschaffen und flächig mit der Abdeckung (**2**) mittels einer zweiten Anhaftung (**40**) verbunden ist.

6. Verfahren nach zumindest einem der Ansprüche 1 bis 5, **dadurch gekennzeichnet, dass** die Erstreckung der Schutzschicht (**4**) vor dem äusseren Rand der Abdeckung (**2**) endet.

7. Verfahren nach zumindest einem der Ansprüche 5 und 6, **dadurch gekennzeichnet, dass** während der Dekontaminationsphase Dekontaminationsmittel durch eine äussere Randzone (**20**) der Abdeckung (**2**), welche nicht von der Schutzschicht (**4**) abgeschirmt ist, auch zu einem Rand (**30**) der ersten Anhaftung (**3**) und auf eine Randfläche (**11**) des Behältnisses (**1**) gelangen kann.

8. Verfahren nach zumindest einem der Ansprüche 1 bis 7, **dadurch gekennzeichnet, dass** als Dekontaminationsmittel Wasserstoffperoxid (H₂O₂) oder Stickstoffdioxid (NO₂) oder ein Begasungsmittel in fachgemässer Konzentration verwendet wird.

9. Verfahren nach zumindest einem der Ansprüche 1 bis 8, **dadurch gekennzeichnet, dass** während der Bereitstellung des Gebindes (**5**) das Behältnis (**1**) mit seinem aseptischen Innenvolumen (**10**) und der Abdeckung (**2**), welche den Durchlass (**12**) verschliesst, in einer beutelartigen Umhüllung (**15**) liegt.

10. Verfahren nach zumindest einem der Ansprüche 1 bis 9, **dadurch gekennzeichnet, dass** die Bereitstellung des Gebindes (**5**) in einem Reinraum (**6**), vorzugsweise mit Reinraumklasse Grade C (gemäss GMP Annex 1: Good Manufacturing Practice), äquivalent zu Klasse 7 (gemäss ISO-Norm 14644-1) erfolgt.

11. Verfahren nach zumindest einem der Ansprüche 9 und 10, **dadurch gekennzeichnet, dass**
a) das Gebinde (**5**) vom Reinraum (**6**) in ein RABS (**7**) transferiert wird und hier die das Gebinde (**5**) umgebende Umhüllung (**15**) entfernt wird; wobei
b) das RABS (**7**) vorzugsweise die Reinraumklasse Grade A für Supply (gemäss GMP Annex 1: Good Manufacturing Practice), äquivalent zu Klasse 5 (gemäss ISO-Norm 14644-1) aufweist.

12. Verfahren nach Anspruch 11, **dadurch gekennzeichnet, dass** die Transfereinrichtung (**8**) einerseits mit dem RABS (**7**) und andererseits mit dem Containment (**9**) verkettet ist.

13. Verfahren zum Transfer eines Gebindes (**5**) zur Verarbeitung in einer Prozesskammer (**90**) eines Containments (**9**) unter aseptischen Bedingungen, in einer *zweiten Ausführungsform,* ist **gekennzeichnet durch** die Abfolge:
a) Bereitstellung des Gebindes (**5**) zumindest umfassend ein Behältnis (**1**) mit einem aseptischen Innenvolumen (**10**), wobei das Behältnis (**1**) ausweist:
aa) einen zuoberst liegenden ersten Durchlass (**12**), der mit einer undurchlässigen Überdeckung (**2**') verschlossen ist; und
ab) einen am Boden oder seitlich des Behältnisses (**1**) liegenden zweiten Durchlass (**12**'), der mit einer partiell durchlässigen Abdeckung (**2**) verschlossen ist;
b) Anbringen einer Schutzschicht (**4**) an der Abdeckung (**2**), wobei die Schutzschicht (**4**):
ba) im Vergleich zur Abdeckung (**2**) weniger für das während einer Dekontaminationsphase auf das Gebinde (**5**) eingeleitete Dekontaminationsmittel durchlässig ist;
bb) sich zumindest partiell oder gänzlich über die Abdeckung (**2**) erstreckt; und
bc) dazu bestimmt ist, dass das während der Dekontaminationsphase auf das Gebinde (**5**) eingeleitete Dekontaminationsmittel nur mehr in einer minimalen, akzeptablen Konzentration **durch** die Abdeckung (**2**) in das Innenvolumen (**10**) gelangt;
c) Durchführen einer Dekontaminationsphase mit dem mit der Abdeckung (**2**) und der Schutzschicht (**4**) versehenen Gebinde (**5**) in einer Transfereinrichtung (**8**);
d) Überführen des mit der Abdeckung (**2**) und der Schutzschicht (**4**) dekontaminierten Gebindes (**5**) in die Prozesskammer (**90**) des Containments (**9**); und
e) in der Prozesskammer (**90**) des Containments (**9**) Entfernen der Überdeckung (**2**') vom Gebinde (**5**), so dass der erste Durchlass (**12**) zum Innenvolumen (**10**) des Behältnisses (**1**) geöffnet ist und die weitere Verarbeitung erfolgen kann.

14. Verfahren nach Anspruch 13, **dadurch gekennzeichnet, dass** die Schutzschicht (**4**):
a) als separates Mittel auf der Abdeckung (**2**) aufgebracht wird; und/oder
b) durch Einwirken auf die Abdeckung (**2**) sich daran eine Strukturänderung bildet, welche als Schutzschicht (**4**) wirkt.

15. Verfahren nach Anspruch 14, **dadurch gekennzeichnet, dass** das Einwirken auf die Abdeckung (**2**) zur Bildung der Schutzschicht (**4**) thermisch oder mittels Bestrahlung, vorzugsweise mit UV-Strahlung oder Ultraschall, geschieht.

16. Verfahren nach zumindest einem der Ansprüche 13 bis 15, **dadurch gekennzeichnet, dass**
a) die Abdeckung (**2**) eine semipermeable Folie ist, z.B. Tyvek^{®}, welche auf einer den zweiten Durchlass (**12**') umlaufenden Randfläche (**11**') des Behältnisses (**1**) mittels einer ersten Anhaftung (**3**) fixiert ist; und
b) die Schutzschicht (**4**) in Gestalt eines separaten Mittels als Folie beschaffen und flächig mit der Abdeckung (**2**) mittels einer zweiten Anhaftung (**40**) verbunden ist.

17. Verfahren nach zumindest einem der Ansprüche 13 bis 16, **dadurch gekennzeichnet, dass** die Erstreckung der Schutzschicht (**4**) vor dem äusseren Rand der Abdeckung (**2**) endet.

18. Verfahren nach zumindest einem der Ansprüche 16 und 17, **dadurch gekennzeichnet, dass** während der Dekontaminationsphase Dekontaminationsmittel durch eine äussere Randzone (**20**) der Abdeckung (**2**), welche nicht von der Schutzschicht (**4**) abgeschirmt ist, auch zu einem Rand (**30**) der ersten Anhaftung (**3**) und auf die den zweiten Durchlass (**12**') umlaufende Randfläche (**11**') des Behältnisses (**1**) gelangen kann.

19. Verfahren nach zumindest einem der Ansprüche 13 bis 18, **dadurch gekennzeichnet, dass** als Dekontaminationsmittel Wasserstoffperoxid (H₂O₂) oder Stickstoffdioxid (NO₂) oder ein Begasungsmittel in fachgemässer Konzentration verwendet wird.

20. Verfahren nach zumindest einem der Ansprüche 13 bis 19, **dadurch gekennzeichnet, dass** während der Bereitstellung des Gebindes **(5)** das Behältnis **(1)** mit seinem aseptischen Innenvolumen **(10)** und der Abdeckung **(2),** welche den zweiten Durchlass **(12')** verschliesst, und der Überdeckung **(2'),** welche den ersten Durchlass **(12)** verschliesst, in einer beutelartigen Umhüllung **(15)** liegt.

21. Verfahren nach zumindest einem der Ansprüche 13 bis 20, **dadurch gekennzeichnet, dass** die Bereitstellung des Gebindes **(5)** in einem Reinraum **(6),** vorzugsweise mit Reinraumklasse Grade C (gemäss GMP Annex 1: Good Manufacturing Practice), äquivalent zu Klasse 7 (gemäss ISO-Norm 14644-1) erfolgt.

22. Verfahren nach zumindest einem der Ansprüche 20 und 21, **dadurch gekennzeichnet, dass**
a) das Gebinde **(5)** vom Reinraum **(6)** in ein RABS **(7)** transferiert wird und hier die das Gebinde **(5)** umgebende Umhüllung **(15)** entfernt wird; wobei
b) das RABS **(7)** vorzugsweise die Reinraumklasse Grade A für Supply (gemäss GMP Annex 1: Good Manufacturing Practice), äquivalent zu Klasse 5 (gemäss ISO-Norm 14644-1) aufweist.

23. Verfahren nach Anspruch 22, **dadurch gekennzeichnet, dass** die Transfereinrichtung **(8)** einerseits mit dem RABS **(7)** und andererseits mit dem Containment **(9)** verkettet ist.
